# EUROPEAN PATENT APPLICATION

(11) **EP 4 198 145 A1**
(43) Date of publication of application: **21.06.2023**
(21) Application number: 21855000.2
(22) Date of filing: 28.05.2021
(51) Int. Cl.: C12Q 1/6844, G01N 21/64, B01L 7/00

(54) **LAMP TESTING DEVICE AND METHOD USING ISOTHERMAL AMPLIFICATION OF RNA/DNA TO IDENTIFY PATHOGENS**

(30) Priority: 14.08.2020 BR 102020016662
(71) Applicant: Fundação Oswaldo Cruz, 21045-900 Rio de Janeiro, RJ (BR)
(72) Inventor: NETO, Rubens, Lima, do Monte, 31320-262 Engenho Nogueira - MG (BR); ALVES, Pedro, Augusto, 31270-720 São Luiz, MG (BR); NEVES, Hércules, Pereira, 30150-020 Belo Horizonte - MG (BR); MARTINS, Henrique, Resende, 31340-025 Belo Horizonte - MG (BR); AVELAR, Bruno, Silveira, 30380-002 Belo Horizonte - MG (BR); FREITAS, Ângelo, Eustáquio, Zandona, 30535-690 Belo Horizonte - MG (BR); RODRIGUES, Denilson, Laudares, 30710-580 Belo Horizonte - MG (BR)
(74) Representative: Zacco Denmark A/S
(86) International application number: PCT/BR2021/050230
(87) International publication number: WO 2022/032364

(57) **Abstract**

The present invention provides a LAMP assay device that promotes isothermal RNA/DNA amplification applied to pathogen identification, comprising: a LAMP assay chamber (1); an electronics cabinet (4), a power supply means (7); and a top lid (2) for closing the LAMP assay chamber (1), wherein internally the device comprises: a metal cylindrical thermoblock (8) comprising openings (80) for positioning microtubes (81); a control board with central processing (14); a power electronics board (13); at least one heating element (10) in contact with the thermoblock (8) and adapted to heat the thermoblock (8) by induction; a temperature sensor (9) adapted to measure the temperature of the thermoblock (8); a plurality of RGB LEDs (12) positioned below the thermoblock (8) and adapted to excite each microtube positioned in the thermoblock (8); and a camera (11) positioned below the thermoblock (8) and adapted to capture images of each of the microtubes (81) positioned in the thermoblock (8). In addition, the invention provides a method for pathogen identification from a LAMP assay device.

## Description

### FIELD OF INVENTION

The present invention brings together a device and methods for rapid molecular diagnosis of diseases. More precisely, it is a platform aimed at pathogen detection through isothermal DNAJRNA amplification, which can be applied to the diagnosis of diseases of human health importance, including COVID-19. It can also be reprogrammed and/or configured for the diagnosis of diseases of veterinary, health surveillance, or agribusiness importance.

### BASIS OF THE INVENTION

The technique of loop-mediated isothermal DNA/RNA amplification, also known as LAMP (*Loop-Mediated Isothermal Amplification*)*,* can be applied in the development of diagnostic tests for the identification of different pathogens. It is a technique for the amplification of nucleic acids using 4 to 6 primers designed to amplify the target sequence by creating *stem-loop* structures that assist in the synthesis of new nucleic acid molecules by DNA polymerase. The LAMP technique takes place at constant temperature (typically ranging from 60 - 65 °C), and because it uses multiple primers to amplify a target sequence, it is also highly specific.

The LAMP technique has been increasingly used as an alternative method for the detection of molecular targets (DNA/RNA), due to its high sensitivity, specificity, reduction of reaction time and the advantages of not requiring a complex laboratory structure to perform the isothermal amplification reaction.

LAMP assays are performed in so-called LAMP assay chambers, or LAMP assay devices as adopted in this report, which allow the amplification of genetic material (DNA or RNA) in samples for pathogen detection and disease diagnosis by heating an assay chamber inside the device.

In these assays, in a summarized way, reagents are added to the biological samples under analysis and the set is heated in a LAMP assay chamber to a given temperature, usually ranging from 60-65 °C. These samples are then monitored during the assay, the result of which can be measured by the alteration of color (or fluorescence) in the reaction, indicating a positive sample, performing real-time readings using displaceable probes to construct a fluorescence vs. time curve, and confirming the presence of the pathogen in question.

Several settings of LAMP assay chambers are known from the state of the art, which will be described in the following paragraphs.

Document CN203720089U describes a fluorescence intensity analyzer of LAMP reactions that comprises a computer data processing device, an optical sensor, a constant temperature heating plate, and a UV light source. The constant temperature heating plate consists of a base and hollow cylinders, where reaction tubes made of light transmissible material are placed. The side walls of the cylinders have holes, on one side aligned with an external ultraviolet light source, and on the other side aligned with an optical sensor, which is connected to a computer data processing device by means of a cable. The sensor is able to compare the intensity of fluorescence emitted by the SYBR Green "dye" - a DNA intercalating fluorophore that fluoresces in the presence of double strands of the molecule - as an indirect measure of the increase in the amount of amplified DNA. In this way it is possible to infer, in real time, about the quantification of a given target (or gene). A useful strategy, for example, to calculate viral loads between samples.

The Nature's article "A smartphone-based diagnostic platform for rapid detection of Zika, chikungunya, and dengue viruses" (Priye et al, 2017) describes the development of a 3D printed device for LAMP assays, combined with a smartphone-based detection system, that enables simultaneous diagnosis of Zika, Dengue, and Chikungunya viruses. The boxshaped device, consists of three primary components: (i) heating module (consisting of a flat heated surface with contact with the tubes of the reaction module); (ii) reaction module; and (iii) a detection module (consisting of a compact LED mounted on the surface, coupled with a multi-pass belt filter to serve as excitation source) and image analysis (the fluorescence signal emitted in the reaction is captured by a smartphone camera and analyzed by a specific application). The three components are connected and controlled remotely via *bluetooth* by a *smartphone* app.

The document "Simultaneous and high sensitive detection of *Salmonella typhi* and *Salmonella paratyphi* a in human clinical blood samples using an affordable and portable device" (Kaur et al, 2019) presents a system for the detection of bacteria in human blood samples based on LAMP, that is composed of a LAMP amplification unit, which consists of multiple cartridge-type heaters and temperature control media, and a detection unit. For detection, SYBR Green dye is added to the sample and the tube is placed in a 3D printed portable device, where there is an LED light source above the tube and a photodetector, positioned at a right angle to the tube, controlled electronically. The device communicates via *bluetooth* with a smartphone using an application that can display, record, and store details of each patient.

Some technical limitations in the operationalization of LAMP assays make it difficult to establish reproducible and highly effective tests. One of these points is related to ensuring homogeneous temperature control inside the reaction tubes. In this regard, the use of chambers employing convection heating or heating plates in contact with only a portion of the surface of the reaction tubes does not provide satisfactory temperature balance in the reaction fluid. Another limiting factor concerns the need for accurate reading of the color change as a result of the reaction. In this aspect, the use of sensors that replace visual inspection is crucial. Furthermore, the type of sensor and its positioning in relation to the reaction tube have a strong influence on the performance of the assay.

In addition to the above, the rapid dissemination of the SARS-CoV-2 virus, the causative agent of the syndrome called COVID-19, has imposed numerous challenges on global public health. Due to the rates of morbidity and mortality observed, the high potential of contagiousness and infectivity of this new coronavirus, besides the virtual impossibility of clinical differential diagnosis in relation to other viral respiratory infections and even other etiologies, it is essential to apply sensitive and differential diagnostic systems.

Thus, the arrival of the Covid-19 pandemic brought with it new challenges in its handling by hospital institutions. Therefore, it is necessary to expand the diagnostic capacity for suspected cases seen in hospitals, whether related to patients or even among healthcare professionals directly exposed.

The exposure of these professionals to the virus during their care, places this professional category as an increased risk for infection, generating a series of medical leave requests due to clinical suspicion, many of them without diagnostic confirmation. This is a serious problem related to personnel management, generating frequent absence in the work teams and configuring itself as a bottleneck in the assistance provided to the population.

As soon as the outbreak of the epidemic in China was reported, several protocols for the detection of SARS-CoV-2 by real-time reverse transcription PCR (RT-PCR) were released. Nevertheless, the development of simpler, cheaper, more agile diagnostic systems that make use of IoT (Internet*of Things*) technology becomes something relevant and very desirable, since the current Brazilian national installed capacity for mass diagnostics and availability of access for users of public health systems, such as SUS, are severely limited.

The main limitations to the implementation of solutions in molecular diagnostics are the high cost, the need for adequate infrastructure, and the training of personnel. Due to the high demand and despite the efforts, there is a huge difficulty in testing suspected cases for SARS-CoV-2 massively lately in Brazil, and also worldwide, both in the public and private sectors. Despite being effective, the RT-PCR technique is offered centrally and there is not enough production of inputs to serve the population, leading the system to prioritize the diagnosis according to severity criteria.

Therefore, in light of the cited state of the art documents, it is noted that the state of the art comprises a number of LAMP assay reaction and reading equipment, with distinct configurations. However, it is clear that many improvements still can be proposed, especially regarding the effectiveness of performing LAMP assays *in situ,* since no single device of LAMP assays has been identified so far as a technical solution to overcome limitations to the control of reaction temperature and accurate color change reading.

Thus, as will be further detailed in the following sections, the present invention aims at presenting a LAMP assay device that comprises constructive features that facilitate its use and make the performance of the tests more efficient.

The invention further discloses an approach based on the LAMP method for disease diagnosis. Illustratively, the method is used for the diagnosis of arboviruses, such as Zika, Dengue, Chikungunya and Yellow Fever, and SARS-CoV-2.

### SUMMARY OF THE INVENTION

The present invention aims to provide a LAMP assay device applied for pathogen identification, which is capable of being applied in remote locations for the diagnosis of Dengue, Zika, Chikungunya, Yellow Fever and SARS.CoV-2, among other pathogens.

The LAMP assays device is controlled by multi-platform software compatible with smartphones, tablets or computers. The application communicates with the device's firmware via wireless connection, the latter being responsible for managing the thermal control, reading the sensors, and automatically interpreting the results through image processing multiplatform software compatible with smartphones, tablets, or computers. The application communicates with the device's firmware via wireless connection, the latter being responsible for managing the thermal control, reading the sensors, and automatically interpreting the results through image processing. Besides being the graphical human-machine interface, the software has a local database, georeferencing, and, when connected to the Internet, automatically establishes communication with a WEB server, capable of centralizing data and compiling reports through *data mining techniques.* There is a risk of leakage of sensitive information, as it is an IoT (Internet of Things) device, in which patient/customer data is sent to the server through an internet connection Thus, the entire system was developed following the General Law of Personal Data Protection (LGPD or LGPDP), Law No. 13.709/2018. Both the Software and the WEB system work under cryptography protocols and security routines that guarantee both data integrity and security.

In order to achieve the described objectives above, the present invention provides a LAMP assay device that promotes isothermal RNA/DNA amplification applied to pathogen identification, comprising: a LAMP assay chamber; a compartment within the cabinet reserved for the proper packaging of the device electronics, a power supply means; and an upper lid for closing the LAMP assay chamber, wherein internally the device comprises: a cylindrical metal thermoblock comprising openings for positioning microtubes; the electronics contains a power electronic board; at least one heating element in contact with the thermoblock and adapted to heat the thermoblock microtubes by induction; a temperature sensor adapted to measure the temperature of the thermoblock; RGB LEDs positioned in the lower part of a darkroom, next to the camera inferiorly and adapted to excite each microtube positioned in the thermoblock; a camera positioned below the thermoblock and adapted to capture images of each of the microtubes positioned in the thermoblock; and a control board with central processing, responsible for performing the processing of the images captured by the camera, controlling the temperature in the thermoblock and performing the communication with a user device (tablet, computer, smartphone, etc.).

In addition, the invention provides a method for identifying pathogens from a LAMP and RT-LAMP assay device as the described one.

### BRIEF DESCRIPTION OF THE IMAGES

The detailed description presented below makes reference to the attached figures and their respective reference numbers.
Figure 1 illustrates a perspective view of an optional setting of the LAMP assay device that promotes isothermal RNA/DNA amplification applied to pathogen identification of the present invention.
Figures 1a, 1b and 1c illustrate front, side and top views, respectively, of the LAMP assay device of figure 1.
Figure 2 illustrates an exploded view of the LAMP assay device setting that promotes isothermal RNA/DNA amplification applied to pathogen identification from figure 1.
Figure 3 illustrates an isolated top view of the thermoblock of figure 2
Figure 3a illustrates a view of the thermoblock from figure 3 with microtubes inserted into the openings for positioning microtubes.
Figure 3b illustrates a sectional view of the thermoblock from figure 3a.
Figure 3c illustrates a sectional view of the thermoblock of figure 3a with a focus on an opening for positioning microtubes.
Figure 4 illustrates an optional setting of the hardware architecture of the LAMP assay device of the present invention.
Figure 5 illustrates the reaction disclosure using specific pH difference color change displaceable probe, according to an experiment performed using the LAMP assay device of the present invention for detection of SARS-CoV-2.
Figure 6 illustrates the strategy for the design of primers F3/B3; FIP/BIP; LF/LB using the N gene of SARS-CoV-2, according to experiments performed.
Figure 7 illustrates a comparison between samples that were positive for SARS-CoV-2.

### DETAILED DESCRIPTION OF THE INVENTION

Preliminarily, it is emphasized that the following description will start from a preferred embodiment of the invention. As it will be evident to anyone skilled in the subject, however, the invention is not limited to this specific embodiment.

Particularly, the use of the device of the present invention for the rapid molecular detection of SARS-CoV-2 RNA will be described in more detail. However, obviously, the invention provides a LAMP assay device that promotes isothermal RNA/DNA amplification applied to the identification of different pathogens, for example, Dengue, Zika, and Chikungunya, among others. It should be noted that in the following paragraphs, for textual simplification purposes, the invention will be referred to simply as the LAMP device, which will certainly not cause confusion to an attentive reader.

Unless otherwise defined, all technical and scientific terms used herein have the same meaning as understood by any person skilled in the art to which the invention pertains. The terminology used in describing the invention is intended to describe particular realizations only, and is not intended to limit the scope of the teachings. Unless otherwise indicated, all figures expressing quantities, percentages and proportions, and other numerical values used in the descriptive report and in claims, are to be understood as being modified in all cases by the term "about". Thus, unless otherwise stated, the numerical parameters shown in the descriptive report and in the claims are approximations that may vary, depending on the properties to be obtained.

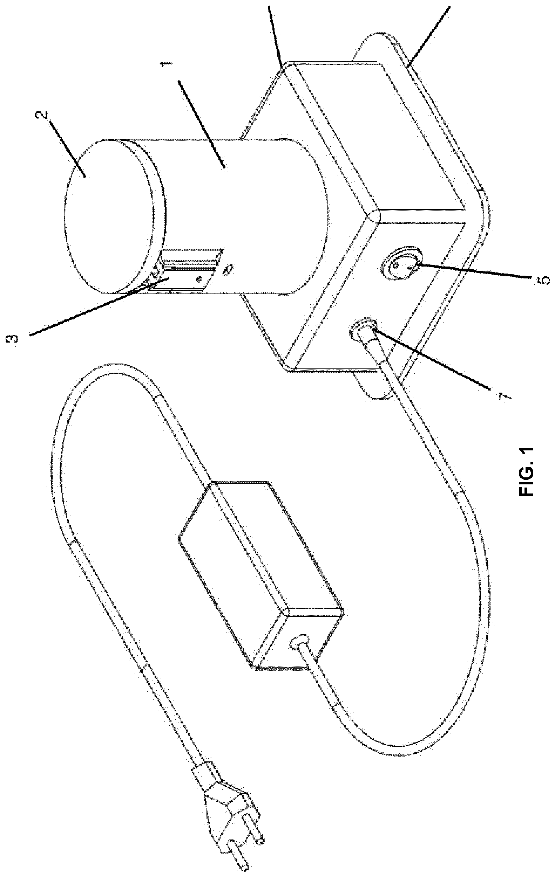

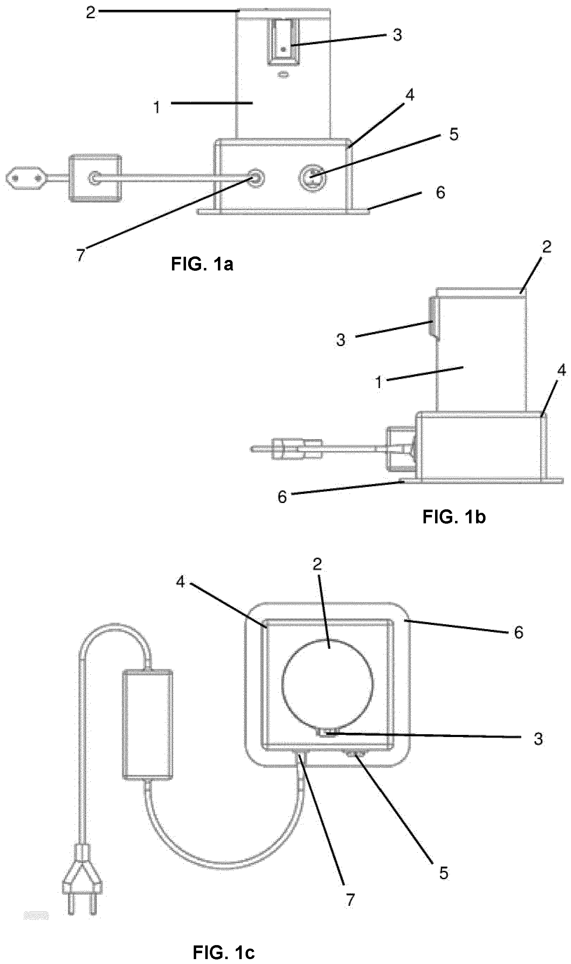

Figure 1 illustrates a perspective view of an optional configuration of the LAMP assay device that promotes isothermal RNA/DNA amplification applied to pathogen identification of the present invention. Figures 1a, 1b and 1c illustrate front, side and top views, respectively, of the LAMP assay device of figure 1.

According to this configuration, the LAMP device externally comprises: a LAMP assay chamber 1; a power source 7 capable of being connected to a power entry point (optionally, a battery can be used); a top lid 2; a bottom lid 6; an electronics cabinet 4, where the electronic control devices are positioned; a sensor 3 for closing the top lid 2; and a button 5 for actuation.

With respect to the power source 7, the LAMP device of the present invention can be connected to an outlet or a mobile battery that provides the power required for its operation, so this feature does not represent a limitation to the scope of the invention.

It is emphasized that the position of the elements indicated in this figure is optional, so that other settings can be adopted, where the more general setting of the present invention will be defined by the main claim, as known to any person skilled in the art.

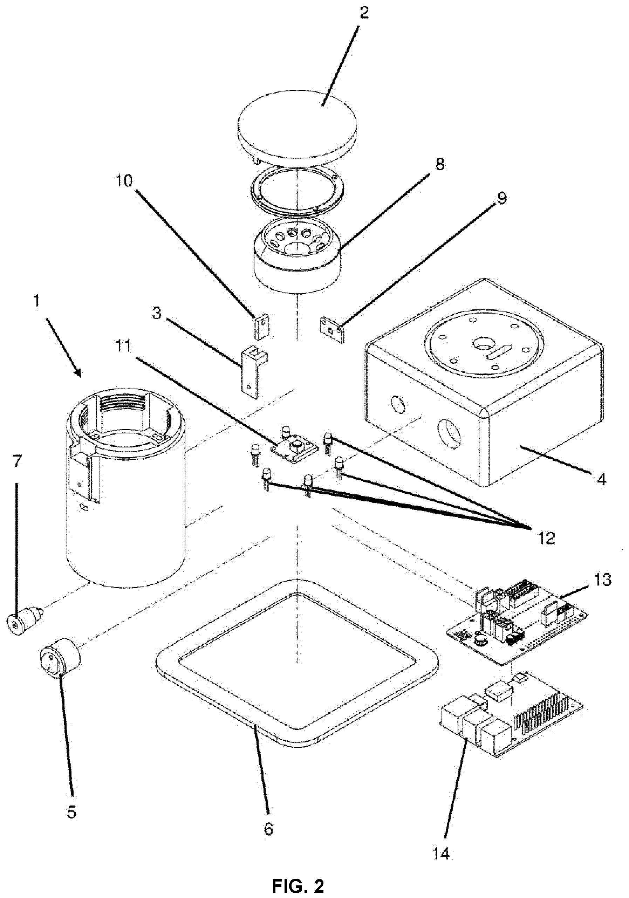

Figure 2 illustrates an exploded view of the LAMP assay device setting that promotes isothermal RNA/DNA amplification applied to pathogen identification from figure 1. Internally the LAMP device comprises: a metal cylindrical microtube thermoblock 8 comprising openings 80 for positioning microtubes 81; a control board with central processing 14; a power electronics board 13; a heating element 10 connected to the thermoblock 8 and adapted to heat the thermoblock 8 by induction; a temperature sensor 9 adapted to measure the temperature of the thermoblock 8; RGB LEDs positioned at the bottom of a darkroom, next to the camera 11 and adapted to excite each microtube positioned in the thermoblock 8; and a camera 11 positioned below the thermoblock (8) and adapted to capture images of each of the microtubes positioned in the thermoblock 8.

In the setting illustrated in the figures of this report, the thermoblock 8 comprises a cylindrical anodized aluminum block with ten openings adapted to fairly accommodate ten 81 microtubes of 0.2 mL each. The cylindrical shape of the thermoblock 8, as well as the tight fit (no gaps) between each microtube and the thermoblock 8, ensure concurrent and uniform heating and homogeneous temperature control inside each of the microtubes 81 positioned in the openings.

Heating takes place as follows, the heater element 10 is in contact with thermoblock 8 and heats thermoblock 8 by induction. Since the thermoblock 8 is metallic (preferably made of anodized aluminum) it is heated quickly and substantially uniformly. Thus, the microtubes are also heated quickly and evenly, as they are tightly fitted to each of the openings 80. Thus, the invention ensures rapid and uniform heating of each of the samples inside the microtubes.

The number of heating elements 10 adopted for the thermoblock 8 may vary in different embodiments of the invention, in particular in dependence on the number of openings 80 for microtubes 81 available in the thermoblock 8. In optional settings of the invention, in which a plurality of openings 80 for microtubes 81 is adopted, more than one heating element 10 may be adopted, distributed in different ways with the aim of providing the fastest and most uniform heating possible of the thermoblock 8, and consequently of the microtubes 81 placed therein.

Thus, in summary, the invention provides for the adoption of at least one heating element 10, and more than one can be adopted (even as many as deemed necessary) according to specific applications, without departing from the scope of protection of the invention.

It should be noted that the resistors of the heating element 10 are controlled electronically by the control board with central processing 14. Thus, once the final temperature to be used in the experiment is programmed, the control board with central processing 14 will control the heating of the support until the temperature sensor 9 identifies that the desired temperature has been reached. From that point on, the control board with central processing 14 will control the heating element 10 to maintain the desired temperature throughout the experiment.

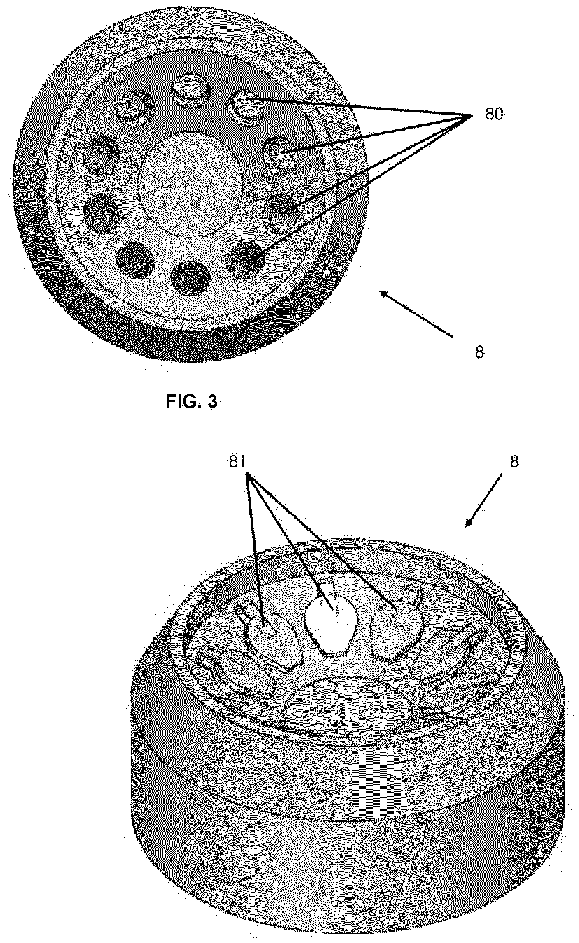

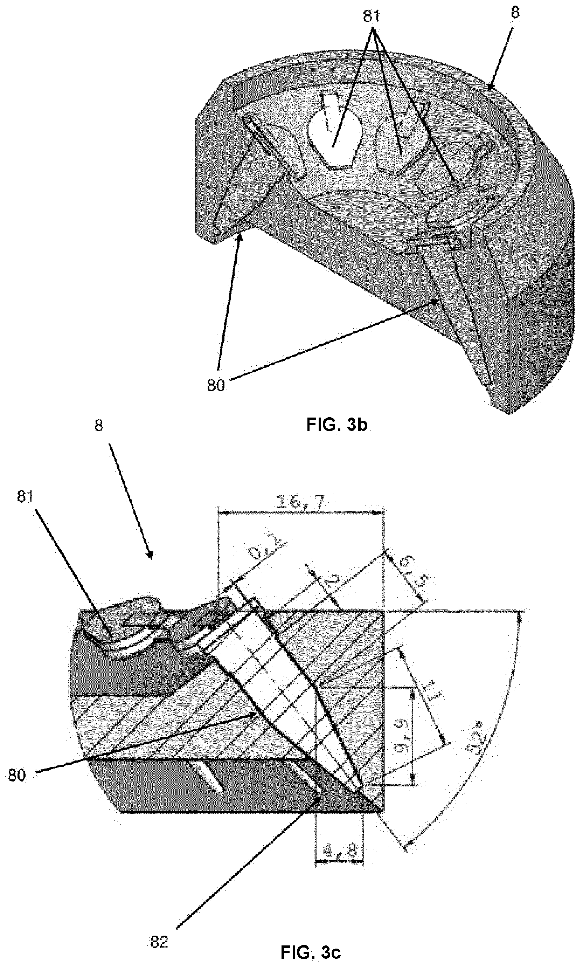

Figure 3 illustrates an isolated top view of the thermoblock 8 from figure 2, figure 3a illustrates a view of the thermoblock 8 from figure 3 with microtubes inserted into the openings 80 for positioning the microtubes, figure 3b illustrates a sectional view of the thermoblock 8 from figure 3a, and figure 3c illustrates a sectional view of the thermoblock from figure 3a with a focus on an opening 80 for positioning microtubes.

The invention further provides that the LAMP reaction promoted using the described LAMP device is combined with a detection method. An example is the detection via alteration of color due to the difference in pH of the positive samples, which become acid - by the liberation of protones during the DNA amplification process - and in the presence of phenol red, turn yellow, different from the negative, nonamplified samples, which present a pinkish shade. In particular examples, the colored dye comprises cresol red, phenol red, m-cresol purple, bromocresol purple, neutral red, naphtholphtalein, thymol blue. In some examples, the pH sensitive indicator dye is a colored dye detectable in visible light. In other examples, the pH-sensitive indicator dye is a fluorescent indicator dye, including but not limited to 2',7-bis-(2-carboxyethyl)-5(6) carboxyfluoresceinna, 5(6)-carboxy-21,71 -dichlorofluorescein, 5(6) carboxyfluorescein, 3 ,6-diacetoxyphthalonitrile, 6,8-dihydroxy-1,3-pyrenedisulfonic acid, or 5-(e-6)-carboxyl seminaftorhodafluor.

It is possible to use other agents that reveal characteristics intrinsic to the reaction, such as the reduction of available magnesium ions by precipitation with the pyrophosphate. Dyes such as hydroxynaphthol blue (HNB) are able to detect this difference in magnesium ions and turn from purple, or dark blue, to sky blue.

This same characteristic of magnesium pyrophosphate precipitation in isotopic amplification reactions results in turbidity of positive samples, which occurs due to an increasing amount of magnesium pyrophosphate precipitate in solution as a byproduct of amplification. Turbidity is measured by visual inspection upon completion of the reaction or in real time using a turbidimeter.

Another way of detection is by chromatography (or migration) on a 2 % agarose gel treated with ethidium bromide or any fluorescent DNA intercalator such as SYBR green or GelRed.

The invention also provides for the use of fluorescent intercalating agents, such as SYBR Green, SYTO 9, SYTO 13, SYTO 16, SYTO 24, SYTO 60, SYTO 62, SYT 64, SYTO 82, SYBR Gold, YOPRO-1, TOTO-1, TOTO-3, BOBO3, POPO3 and TOPRO3 (Invitrogen), Eva Green (Biotium), Boxto (TATA Biocentre), Miami Green, Miami Yellow, Miami Orange (Kerafast), Pico 488 (Lumiprobe), malachite green, without necessarily using agarose gel band resolution

In a further embodiment, the invention comprises the use of displaceable fluorescent probes for detection, which bind with high specificity to their targets. Thus, the camera 11 positioned inferiorly to the thermoblock 8 is adapted to capture the fluorescence emitted by the probes present in the amplification reaction. Examples of fluorescent dyes that may be used in the invention include, for example, calcein, Mag-Fura-2, and Magnesium green (Life Technologies, Grand Island, N.Y.) and Fluo-2Mg, Fura-2Mg.Indo-1 Mg, and Asante Magnesium green (TEF Labs, Austin, Texas). Fluorescence is detectable using an instrument, such as a fluorimeter that may be associated with a real-time PCR thermal cycler, making up a single system.

To allow accurate reading of the reaction result, as illustrated in figures 3, 3a, 3b, and 3c preferably, the openings 80 for positioning microtubes 81 are angled at an angle of approximately 45° (can vary substantially between 38° and 52°, as illustrated in figure 3c).

It should be noted that the dimensions illustrated in figure 3c represent only an optional setting of a realized prototype of the invention, whereby these specific dimensions may vary according to the embodiment performed. Thus, the invention is not limited to that specific embodiment illustrated in that figure.

The thermoblock 8 further comprises an opening 82 in the lower inner portion of each opening 80 for positioning microtubes 81. Thus, through opening 82, a light is allowed to be excited with four fluorescence signal bands (or light channels from RGB LEDs) available, and it is also possible for the camera 11 to capture the image of each microtube.

The reading of fluorescence in different bands enables the concurrent detection of different targets and the quantification of the charge present in the sample, by using different probes labeled in the same reaction.

Optionally, a control board with central processing in which the processor is a 64-bit QuadCore 1.4GHz ARMV8 (code: BCM2837B0) was adopted due to the simplicity of programming for a prototype in which the invention was tested. However, any other control board with central processing can be adopted to perform the functions defined in this report.

It is important to point out that the control board with central processing (14) is responsible for managing the entire performance of the assays, namely: perform the processing of the images captured by camera 11; control the temperature inside the assay chamber 1 LAMP, perform the automatic detection of the assay through image processing, and perform the communication with a graphical interface software compatible with tablet, computer, smartphone, etc.

Preferably, the communication between the control board with central processing 14 and the user device is performed by wireless technology, preferably bluetooth. However, in alternative settings, communication can be accomplished in different ways including wireless ways, or with cables.

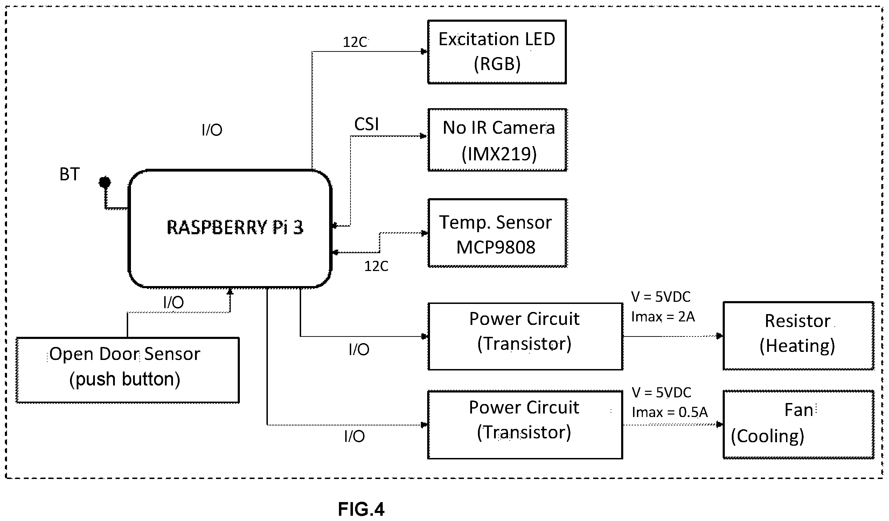

Figure 4 illustrates an optional setting of the hardware architecture of the LAMP device of the present invention.

Thus, in a more broadly way, the LAMP assay device that promotes isothermal RNA/DNA amplification applied to pathogen identification of the present invention comprises: a LAMP assay chamber 1; an electronics cabinet, a power supply means 7; and a top lid 2 for closing the LAMP assay chamber 1, internally the device comprises: a cylindrical metal thermoblock 8 comprising openings 80 for positioning microtubes 81; a control board with central processing 14; a power electronics board 13; at least one heating element 10 in contact with the thermoblock 8 and adapted to heat the thermoblock 8 by induction; a temperature sensor 9 adapted to measure the temperature of the thermoblock 8; a plurality of RGB LEDs 12 positioned below the thermoblock 8; and a camera 11 positioned below the thermoblock (8) adapted to capture images of each of the microtubes positioned in the thermoblock 8. According to this more general setting, the control board with central processing (14) further comprises means for communicating with a mobile user device (such as a smartphone, a computer, a tablet, etc.) and is adapted to control the temperature of the thermoblock 8 according to a schedule established by controlling the heating element 10 and in response to the signal of the temperature sensor 9. It is also adapted to perform the processing of the images captured by camera 11.

Optionally, there is a visual operation warning element to inform a user about the status of the device. Preferably, this element is an LED positioned externally to the LAMP device to indicate the condition of the device.

The device can further adopt a lid-closing sensor 3 in communication with the central processing control board (14), where the central processing control board (14) is adapted to allow the device to operate only when the top lid 2 is closed. The lid-closing sensor can be any that is known from the state of the art, so this does not represent a limitation to the scope of the invention.

Thus, for the use of the described device, the top lid 2 is opened to allow microtubes 81 to be inserted into each opening 80 to position microtubes 81 from thermoblock 8. As described, the number of openings 80 for positioning microtubes 81 can vary in different settings of the invention. The specific model illustrated in this report adopts a total of ten openings. However, as already described, the number of openings can vary in different embodiments of the invention, without departing from the scope of protection of the claims.

After closing the top lid 2, the lid-closing sensor 3 unlocks the operation of the device that can proceed with the assay. If the lid is not properly closed, an error signal/alert can be sent for the user to correct the problem (close the lid properly). This signal can be a visual signal on the device itself, or a message (visual and/or audible and/or by vibration of the user device) can be sent to the user device informing the user of the identified problem.

From this point on, the user interaction with the LAMP device takes place via the user device that is in communication with the control board with central processing (14). Then the user selects the diagnostic reaction to be performed (Dengue, Zika, Chikungunya, Yellow Fever, Covid-19, etc) through an application on the user device, which will start a process that contains the following steps:
activate at least one heating element 10 to raise the temperature of the thermoblock 8 to a preset temperature for the defined diagnostic reaction;
maintain the temperature of the thermoblock 8 at the preset temperature for a preset period of time for the defined diagnostic reaction, and turn off the heating element 10;
turn on the plurality of RGB LEDs 12 for the excitation of each microtube (sample) positioned in the thermoblock 8;
capture the values with camera image 11 for each position of each microtube and for each of the excitation colors, and send this information to the user device;
process the information received by the user device, which involves the conversion of image information from RGB to chrominance and check in which region of the 2D plane each value is located;
define a positive or negative diagnosis for each microtube (sample) from the processing of the information;
perform real-time readings using displaceable probes to construct a fluorescence vs time curve (optional);
display the diagnostic result of each microtube through the user device;
store the diagnostic result of each microtube in a local database (optional); and
send the diagnostic result of each microtube to a database in the cloud (optional).

More specifically, the step of capturing values with camera image 11 for each microtube, and for each of the excitation colors, can further include using the green, red, and orange fluorophores, for example.

At the end of the process, it is also possible that a cooling device (optionally a fan) is activated to cool down assay chamber 1 of the LAMP device.

From what has been described so far, and in view of the recent developments related to the COVID-19 pandemic the world faces, experiments were conducted in which the LAMP device of the invention was tested to verify its applicability in performing assays for the identification of those contaminated by COVID-19.

In this sense, the use of the Loop Mediated Isothermal Amplification (LAMP) technique for rapid molecular detection of nucleic acids in biological samples has been proposed.

The LAMP technique is characterized by its high specificity and sensitivity that generates amplified nucleic acids in a short interval of time by using a DNA polymerase with chain shift activity (Notomi et al., Nucl. Acids. Res. 28:E63, 2000). LAMP can also be used to amplify a target RNA molecules by adding reverse transcriptase (RT) in a single-step reaction by using constant temperature, and is called RT-LAMP. This methodology is used to diagnose pathogens from biological samples. For example, these pathogens can be Zika, Dengue, Chikungunya, Yellow Fever, and SARS.CoV-2, among others.

In one embodiment, this technique can be used for molecular detection of SARS-CoV-2 RNA in biological samples, preferably from oropharyngeal and nasopharyngeal swabs and saliva, sputum, bronchoalveolar lavage, etc.

In this regard, the biological samples to which the present invention refes to are the oropharyngeal and nasopharyngeal swabs, saliva, bronchoalveolar lavage, among others containing nucleic acids, which can be isolated from the group comprising, but not limited to: cells, tissues, blood, serum, plasma, saliva, urine, cerebrospinal fluid, nasopharyngeal aspirates, middle ear fluids, bronchoalveolar lavage, tracheal aspirates, sputum, vomit, buccal swab, vaginal swab, rectal swab, and feces. The samples can be used directly in the amplification reaction. In another embodiment, the samples are first treated with lysis buffer or heat treated, prior to their addition to the reaction means. The methodologies for isolating/extracting nucleic acids from samples are widely known to any person skilled in the art.

The LAMP-Loop mediated isothermal amplification technique employs a set of at least 4 four primers (2 pairs, an inner pair and an outer pair) that bind to six distinct regions within the target molecule. Forward inner primers (FIP) and Backward Inner Primer (BIP) have two distinct annealing regions: the F2 region, in the case of FIP, and B2 region, in the case of BIP, are located in the 3' portion of the primers and pair directly with the target molecule; the Flc and Blc regions are sequences complementary and inverted to an internal region of the target molecule strand that will be polymerized by the F2 and B2 regions, respectively. The external primers, F3 and B3, are externally paired to the internal primers, so that their function is to serve as an anchor point for the DNA polymerase to invade the strand formed in the polymerization initiated by the F2/B2 region. In some embodiments, two more primers (2 pairs) that bind to the loop can be used (LoopF or LF - Forward loop primer, and LoopB or LB - Backward loop primer), making a total of 6 primers and 8 binding regions.

For purposes of this invention, "primer" refers to an enzymatically extendable oligonucleotide comprising a defined sequence that is designed to hybridize in an antiparallel way with a complementary portion of a target nucleic acid sequence. Therefore, the primer is usually provided in molar excess relative to its target nucleic acid sequence. A primer does not need to be 100% complementary to its target for the elongation to occur; primers with less than 100% complementarity may be sufficient for hybridization and enzyme for the elongation to occur. A primer is preferably, but not necessarily, synthetic, and is usually about 10 to about 100 nucleotides in length. In addition, for purposes of this invention, sequences with at least about 85%, more preferably at least about 90%, 95%, 96%, 97%, 98% or 99% of identity with the primers described herein are included, as measured by well-known sequence identity assessment algorithms such as FASTA, BLAST or Gap.

Unlike the PCR technique, in the LAMP technique the use of a thermocycler is not necessary and the reaction preferably takes place at a constant temperature, most preferably the temperature is between 60¬70°C, and even more preferably 60-65°C The reaction time can vary between 10 and 120 minutes, more preferably between 15 and 90 minutes, even more preferably between 20 and 60 minutes.

The DNA polymerase used is selected from the group consisting of DNA polymerase Bst, DNA polymerase Bsm, DNA polymerase Gsl and DNA polymerase SD and combinations thereof. In particular, the DNA polymerase enzyme is DNA polymerase Bst.

The reverse transcriptase (RT) used in the RT-LAMP reaction can be any RT enzyme appropriate for the assay, which can be selected by the person skilled in the art. In some embodiments, the RT enzyme is from avian myeloblastosis virus (AMV) or Moloney murine leukemia virus (MMLV).

It is worth noting that the LAMP device of the invention meets point-of-care (POC) criteria and may be useful for the decentralization and democratization of molecular diagnosis of COVID-19.

Furthermore, the use of inputs for molecular diagnosis of COVID-19, unlike those used for the RT-PCR technique, takes the focus off an international dispute and favors diversification in the search for alternative solutions, giving a chance for the test to reach those who need it most, the patient or health professional suspected of contamination.

For detection purposes, probes and primers can include a detectable label, such as a radiolabel, fluorescent dye, biotin, enzyme, or chemiluminescent compound, where the label can be provided before, during, or after hybridization of the probe or primer to the target sequence or its complement.

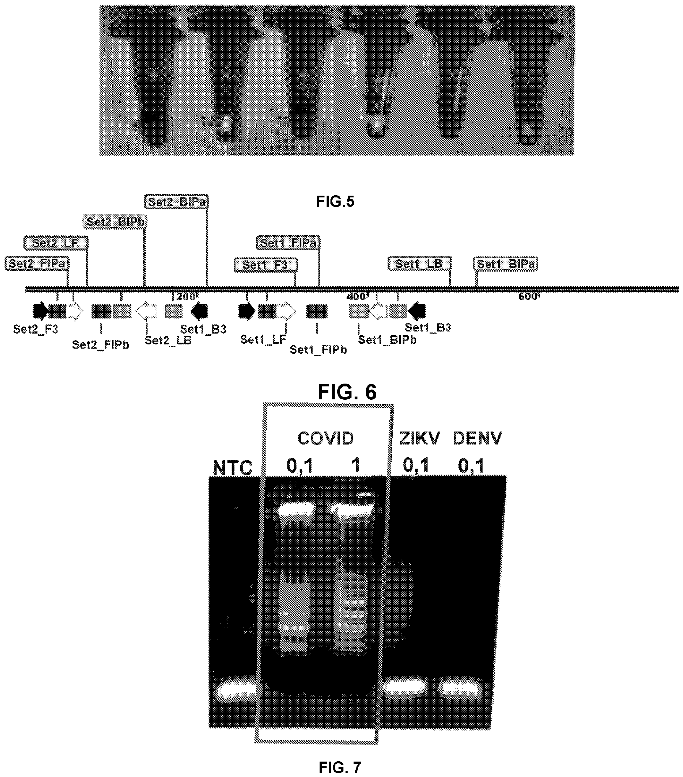

For purposes of this invention, the term "probe" is a nucleic acid molecule that has, attached to it, a detectable label or reporter molecule. The probe size is, for example, at least 10 nucleotides or more in length, such as 10-60, 15-50, 20-40, 20-50, 25-50, 30-60 nucleotides. The detectable labels or reporter molecule may comprise fluorescent and fluorogenic labels (e.g., fluorophores), chromogenic portions, haptenes (such as biotin, digoxigenin, and fluorescein), affinity labels, and radioactive isotopes (such as 32P, 33P, 35S, and 1251). The label may be directly detectable (e.g. optically detectable) or indirectly detectable (e.g. through interaction with one or more additional molecules that are, in turn, detectable). For this application, the technique of displaceable probes, in which the LF primer is functionalized with fluorescent (fluorophore) labels, whose output is specific to the selected target with higher sensitivity than RT-PCR, may be used. The result of this test can also be verified by the migration of the reaction product, illustrated in figure 5 which shows the image of six reaction microtubes using a specific displaceable probe of pH difference color change where the two tubes on the right of the image have different colors and that such differences can be detected by the device's image processing.

In a preferred embodiment of the present invention, the labels include a fluorescent and a quencher portion.

The fluorescent fraction emits light energy at a specific emission wavelength when excited by light energy at an appropriate excitation wavelength. When the fluorescent portion and the quencher portion are held in close proximity, the light energy emitted by the fluorescent portion is absorbed by the quencher portion. But when a probe hybridizes with the nucleic acid present in the sample, the fluorescent and quencher portions are separated from each other by the action of DNA polymerase Bst and the light energy emitted by the fluorescent portion can be detected. Fluorescent portions that are excited and emit at different, distinguishable wavelengths can be combined with different probes. Different probes can be added to a sample, and the presence and amount of target nucleic acids associated with each probe can be determined by alternately exposing the sample to light energy at different excitation wavelengths and measuring the light emission from the sample at the different wavelengths corresponding to the different fluorescent portions.

The fluorophores that can be used in the probes and primers disclosed herein can be selected from the group comprising: acridine and acridine isothiocyanate, 4-amino-N [3-vinyl-sulfonyl) phenyl] naphthalimide-3,5 disulfonate ( Lucifer Yellow VS); Brilliant Yellow; coumarin and derivatives such as 7-amino-4-methylcoumarin (AMC, Coumarin 120), 7-amino-4-trifluoromethylcouluarin (Coumaran 151); cyanine; 4',6-diaminidino-2-phenylindole (DAPI); etidium; -fluorescein and derivatives such as 5-carboxyfluorescein (FAM), 2'7'-dimethoxy-4'51-dichloro-6-carboxyfluorescein (JOE), fluorescein, 6-carboxy-fluorescein (HEX) and TET ( tetramethyl fluorescein); Phenol red; rhodamine and derivatives such as 6-carboxy-X-rodamine (ROX), rhodamine (Rhod), rhodamine B, rhodamine 123, rhodamine X isothiocyanate, N, N, N 'N'-tetramethyl-6-carboxyrodamine (TAMRA); sulforodamine B; sulforodamine 101 and sulforodamine 101 sulfonyl chloride derivative (Texas Red); LightCycler Red 640; Cy5.5; and Cy56-carboxyfluorescein; other fluorophores include Cy3; CyS, VIC (from Applied Biosystems); LC Red 640; LC Red 705; and Quasar^{®} 570, Quasar^{®} 670, CalRed 590, CalRed 610, CalRed 615, CalRed 635, CalGreen 520, CalGold 540 and CalOrange 560 (Biosearch Technologies, Novato, California).

The present invention also includes diagnostic kits. As described herein, the diagnostic kit of the invention is based on loop-mediated isothermal amplification assay (LAMP) to amplify a target region within a target pathogen, comprising, but not limited to Zika, Dengue, Chikungunya, Yellow Fever and SARS-CoV, in a sample, wherein the kit comprises:
(1) a mixture of primers designed to amplify a pathogen sequence by loop-mediated isothermal amplification assay, thereby producing at least one reaction product.

In an exemplary way, primers are those described in SEQ ID NOS: 1-6, or sequences with at least 80%, 85%, 90%, 91%, 92%, 93%, 94%, 95%, 96%, 97%, 98%, or 99% of identity to them.

In another exemplary way, the primers are those described in SEQ ID NOS: 7-12, or sequences with at least 80%, 85%, 90%, 91%, 92%, 93%, 94%, 95%, 96%, 97%, 98%, or 99% of identity to them.

In another exemplary way, the primers are those described in SEQ ID NOS: 13-18, or sequences with at least 80%, 85%, 90%, 91%, 92%, 93%, 94%, 95%, 96%, 97%, 98%, or 99% of identity to them.

In another exemplary way, the primers are those described in SEQ ID NOS: 19-24, or sequences with at least 80%, 85%, 90%, 91%, 92%, 93%, 94%, 95%, 96%, 97%, 98%, or 99% of identity to them.

In another exemplary way, the primers are those described in SEQ ID NOS: 25-30, or sequences with at least 80%, 85%, 90%, 91%, 92%, 93%, 94%, 95%, 96%, 97%, 98%, or 99% of identity to them.

In another exemplary way, the primers are those described in SEQ ID NOS: 31-36, or sequences with at least 80%, 85%, 90%, 91%, 92%, 93%, 94%, 95%, 96%, 97%, 98%, or 99% of identity to them.

In another exemplary way, the primers are those described in SEQ ID NOS: 37-42, or sequences with at least 80%, 85%, 90%, 91%, 92%, 93%, 94%, 95%, 96%, 97%, 98%, or 99% of identity to them.

In another exemplary way, the primers are those described in SEQ ID NOS: 43-48, or sequences with at least 80%, 85%, 90%, 91%, 92%, 93%, 94%, 95%, 96%, 97%, 98%, or 99% of identity to them.

In another exemplary way, the primers are those described in SEQ ID NOS: 49-54, or sequences with at least 80%, 85%, 90%, 91%, 92%, 93%, 94%, 95%, 96%, 97%, 98%, or 99% of identity to them.

In another exemplary way, the primers are those described in SEQ ID NOS: 55-60, or sequences with at least 80%, 85%, 90%, 91%, 92%, 93%, 94%, 95%, 96%, 97%, 98%, or 99% of identity to them.

In another exemplary way, the primers are those described in SEQ ID NOS: 61-66, or sequences with at least 80%, 85%, 90%, 91%, 92%, 93%, 94%, 95%, 96%, 97%, 98%, or 99% of identity to them.

In another exemplary way, the primers are those described in SEQ ID NOS: 67-72, or sequences with at least 80%, 85%, 90%, 91%, 92%, 93%, 94%, 95%, 96%, 97%, 98%, or 99% of identity to them.

The primer sets are supplied in one or more containers, microtubes, multiwell plates, or cards. The primers can be provided suspended in an aqueous solution or as a lyophilized powder, for example.

In another form of embodiment, the kit may further comprise, in addition to (1) above:
(2) a nucleic acid probe comprising a polynucleotide of at least 10 nucleotides in length that is sufficiently complementary to the amplified region, in such way that the nucleic acid probe and the amplification product are hybridisable, where the probe is conjugated with a detectable label or reporter molecule.

Preferably, the probe comprises the primer LF (or LB), or a sequence with at least 80%, 85%, 90%, 91%, 92%, 93%, 94%, 95%, 96%, 97%, 98% or 99% with such, functionalized with a detectable label or reporter molecule.

In another form of embodiment, the kit may further comprise, in addition to (1) above:
(2') includes a pH sensitive indicator dye.

In some embodiments, the kit still includes one or more components for performing the LAMP or RT-LAMP reaction, for example, buffer, DNA polymerase, reverse transcriptase, dNTPs, or any combination thereof.

One or more primers or nucleic acids used as a positive and/or negative control can be part of the kit. Examples of negative controls include any nucleic acids from pathogens other than those that are detected by the kit (e.g., non-SARS-Cov-2 nucleic acid for a kit for detection of SARS-Cov-2, a non-zika nucleic acid for a kit for detection of zika virus, and so on).

The following examples are provided to illustrate particular embodiments. These examples should not be understood as limiting to the invention.

### EXAMPLES

### Example 1: Exemplary primers of the invention

As a target for nucleic acid detection in biological samples, the present invention illustratively discloses the primers from tables 1 to 10.

**Table 1 - Zika Primers**

| iniciador | Sequências |
|---|---|
| Set1_F3 | GCAGAGCAATGGATGGGATA (SEQ ID NO: 1) |
| Set1_B3 | CCCATCCTTGAGGTACAGCT (SEQ ID NO: 2) |
| Ser1_FIP | AACCTGAGGGCATGTGCAAACCGCGGTCAGTGGAGATGACT (SEQ ID NO: 3) |
| Set1_BIP | CACAGGAGTGGAAACCCl'CGACrGAAGTGGTGGGAGCAGAA (SEQ ID NO: 4) |

| Iniciador | Sequências |
|---|---|
| Set1_LF | TCGATTGGCTTCACAACGC (SEQ ID NO: 5) |
| Set1_LB | GGAGCAATTGGGAAGAAGTCC (SEQ ID NO: 6) |

**Table 2 - Dengue Primers**

| Iniciador | Sequências |
|---|---|
| Set1_F3 | GCTGTACGCACGGTGTAG (SEQ ID NO: 7) |
| Set1_B3 | CCTGGAA1GATGCTGAGGAG (SEQ ID NO: S) |
| Setl_FIP | GGTACAGCTTCCCTCAGTGCTCGTGGTTAGAGGAGACCCCT (SEQ ID NO: 9) |
| Set1_BIP | AGAGGTTAGAGGAGACCCCCCAGCAGGATCTCTGGTCTCTC (SEQ ID NO: 10) |
| Set1_LF | CTGCTGCGTTGTGTCAT (SEQ ID NO: 11) |
| Set1_LB | CAGCATATTGACGCTGG (SEQ ID NO: 12) |

**Table 3 - Chikinuninva Primers**

| Iniciador | Sequências |
|---|---|
| Set1_F3 | CGTCAACGTACTCCTAAC (SEQ ID NO: 13) |
| Sat1_B3 | ACGTTGGCTTTRTTTIGG (SEQ ID NO: 14) |
| Set1_FIP | GAAGTTTCCTTTCGGTGGGTTTTTGGAAGACACTYTCYGG (SEQ ID NO: 15) |
| Set1_BIP | AAGGAGTGGGAGGTGGATTTTTTCAYTTGGTGACTGCAG (SEQ ID NO: 16) |
| Setl_LF | AGCGTCTTTATCCACGGG (SEQ ID NO: 17) |
| Set1_LB | AYGCATCRATAATGGCGGG (SEQ ID NO: 18) |

**Table 4 - Yellow Fever Primers**

| Iniciador | Sequências |
|---|---|
| Set1_F3 | TCCACACCTTGGAGGCATTA (SEQ ID NO: 19) |
| Set1_B3 | GTCCATCACAGTTGCCATCA (SEQ ID NO: 20) |
| Set1_FIP | GGCCTCCGATTGATCTCGGCGTGTGAGTGGCCACTGAC (SEQ ID NO: 21) |
| Set1_BIP | GGTTCAGACGAACGGACCTTGGCCCTGGGCAAGCTTCTCT (SEQ ID NO: 22) |
| Set1_LF | CTTCAACTGATGTTCCAATCGTATG (SEQ ID NO: 23) |
| Set1_LB | ATGCAGGTACCACTAGAAGTGA (SEQ ID NO: 24) |

**Table 5 - N gene SARS-CoV2 Primers**

| Iniciador | Sequências |
|---|---|
| SARSCov2_ gNSet1_F3 | TGGCTACTACCGAAGAGCT (SEQ ID NO: 25) |
| SARSCov2_ gNSet1_B3 | TGCAGCATTGTTAGCAGGAT (SEQ ID NO: 26) |
| SARSCov2_ gNSet1_FIP | TCTGGCCCAGTTCCTAGGTAGrGACGAATTCGTGGTGGTGA (SEQ ID NO: 27) |
| SARSCov2_ gNSet1_BIP | AGACGGCATCATATGGGTTGCACGGGTGCCAATGTGATCT (SEQ ID NO: 28) |
| SARSCov2_ gNSet1_LF | TGGACTGAGATCTTTCATTTTACCG (SEQ ID NO: 29) |
| SARSCov2_ gNSet1_LB | ACTGAGGGAGCCTTGAATACA (SEQ ID NO: 30) |
| SARSCov2_ gNSet2_F3 | TGGACCCCAAAATCAGCG (SEQ ID NO: 31) |
| SARSCov2_ gNSet2_B3 | GCCTTGTCCTCGAGGGAAT (SEQ ID NO: 32) |
| SARSCov2_ gNSet2_FIP | CCACTGCGTTCTCCATTCTGGTAAATGCACCCCGCATTACG (SEQ ID NO: 33) |
| SARSCov2_ gNSet2_BIP | CGCGATCAAACAACGTCGGCCCTTGCCAATGTTGAGTGAGA (SEQ ID NO:34) |
| 5ARSCov2_ gNSet2_LF | TGAATCTGAGGGTCCACCAAA (SEQ ID NO: 35) |
| SARSCov2_ gNSet2_LB | CCTTTACCCAATAATACTGCGTCTT (SEQ ID NO: 36) |

**Table 6 - E gene SARS-CoV2 Primers**

| Iniciador | Sequências |
|---|---|
| SARSCov2_ gE_Set1_F3 | TGATGAGCCTGAAGAACATG (SEQ ID NO: 37) |
| SARSCov2_ gE_Set1_B3 | CGCTATTAACTATTAACGTACCT (SEQ ID NO: 38) |
| SARSCov2_ gE_Set1_FIP | TCGGTTCATCATAAATTGGTTCCATCAAATTCACACAA TCGACGG (SEQ ID NO: 39) |
| SARSCov2_ gE_Set1_BIP | ACGACTACTAGCGTGCCTTTGTCTCTTCCGAAACGAA TG (SEQ ID NO: 40) |
| SARSCov2_ gE_Set1_LF | ACTGGATTAACAACTCCGGATGA (SEQ ID NO: 41) |
| SARSCov2_ gE_Set1_LB | GTAAGCACAAGCTGATGAGTACGAA (SEQ ID NO: 42) |
| SARSCov2_ gE_Set2_F3 | TTGTAAGCACAAGCTGATG (SEQ ID NO: 43) |
| SARSCov2_ gE_Set2_B3 | AGAGTAAACGTAAAAAGAAGGTT (SEQ ID NO: 44) |
| SARSCov2_ gE_Set2_FIP | CGAAAGCAAGAAAAAGAAGTACGCTAGTACGAACTT ATGTACTCATTCG (SEQ ID NO: 45) |
| SARSCov2_ gE_Set2_BIP | TGGTATTCTTGCTAGTTACACTAGCAAGACTCACGTT AACAATATTGC (SEQ ID NO: 46) |
| SARSCov2_ gE_Set2_LF | ACGTACCTGTCTCTTCCGAAA (SEQ ID NO: 47) |
| SARSCov2_ gE_Set2_LB | CATCCTTACTGCGCTTCGATT (SEQ ID NO: 48) |

**Table 7 - RdRn gene SARS-CoV2 Primers**

| niciador | Sequências |
|---|---|
| SARSCov2_RdRp_Set1_F3 | CTGTCAAATTACAGAATAATGAGC (SEQ ID NO: 49) |
| SARSCov2_RdRp_Set1_B3 | TCCATCACTCTTAGGGAATC (SEQ ID NO: 50) |
| SARSCov2_RdRp_Set1_FIP | TGTCATCAGTGCAAGCAGTTTGCTGTTGCACTACGACAGA (SEQ ID NO: 51) |
| SCov2_RdRp_Set1_BIPA | CGTTAGCTTACTACAACACACCCCATTTCAAATCCTGTAAATCG (SEQ ID NO: 52) |
| ov2_RdRp_Set1_LFAC | CAGCACAAGACA (SEQ ID NO: 53) |
| 2_RdRp_Set1_LBAAAG | GAGGTAGCTTTGTACT (SEQ ID NO: 54) |

**Table 8 - N2 gene SARS-CoV2 Primers**

| Iniciador | Sequências |
|---|---|
| N2_F3 | ACCAGGAACTAATCAGACAAG (SEQ ID NO: 55) |
| N2_B3 | GACTTGATCTTTGAAATTTGGATCT (SEQ ID NO: 56) |
| N2_FIP | TTCCGAAGAACGCTGAAGCGGAACTGATTACAAACATTGGCC (SEQ ID NO: 57) |
| N2_BIP | CGCATGCATGGAAGTCACAATTTGATGGCACCTGTGTA (SEQ ID NO: 58) |
| N2_LF | GGGGGCAAATTGTGCAATTTG (SEQ ID NO: 59) |
| N2_LB | CTTCGGGAACGTGGTTGACC (SEQ ID NO: 60) |

**Table 9 - El gene SARS-CoV2 Primers**

| Iniciador | Sequências |
|---|---|
| E1_P3 | TGAGTACGAACTTATGTACTCAT (SEQ ID NO: 61) |
| E1_B3 | TTCAGATTTTTAACACGAGAGT (SEQ ID NO: 62) |
| E1_FIP | ACCACGAAAGCAAGAAAAAGAAGTTCGTTTCGGAAGAGACAG (SEQ ID NO: 63) |
| E1_B1P | TTGCTAGTTACACTAGCCATCCTTAGGTTTTACAAGACTCACGT (SEQ ID NO: 64) |
| E1_LF | CGCTATTAACTATTAACG (SEQ ID NO: 65) |
| E1_LB | GCGCTTCGATTGTGTGCGT (SEQ ID NO: 66) |

**Table 8 - As le gene SARS-CoV2 Primers**

| Iniciador | Sequências |
|---|---|
| As1e_F3 | CGGTGGACAAATTGTCAC (SEQ ID NO: 67) |
| As1e_B3 | CTTCTCTGGATTTAACACACTT (SEQ ID NO: 68) |
| As1e_FIP | TCAGCACACAAAGCCAAAAATTTATTTTTCTGTGCAAAGG AAATTAAGGAG (SEQ ID NO: 69) |
| As1e_BIP | TATTGGTGGAGCTAAACTTAAAGCCTTTTCTGTACAATCC CTTTGAGTG (SEQ ID NO: 70) |
| As1e_LF | TTACAAGCTTAAAGAATGTCTGAACACT (SEQ ID NO: 71) |
| As1e_LB | TTGAATTTAGGTGAAACATTTGTCACG (SEQ ID NO: 72) |

### Example 2: COVID-19 Diagnosis

In one example embodiment, the methodology disclosed by the present invention uses as targets for detection of SARS.CoV-2 RNA two regions of the N gene that encodes for the nucleoprotein, as illustrated in Figure 6. This target has proven to be specific and does not react with the RNA from other viruses as it was clear from the tests performed (figure 7).

However, it is also anticipated the utilization of other targets for detection of SARS-Cov-2 RNA, such as the gene encoding for the E and N2 proteins, and part of the lab ORF (NSP3, RdRp, As le).

The LAMP methodology with viral RNA is performed by means of amplification reactions by using the enzyme Bst 3.0 DNA Polymerase (New England Biolabs) or with a mixture containing Bst 2.0 DNA Polymerase WarmStart and RTx (New England Biolabs; in a reaction of 25 IttL of the final volume, containing the primers FIP, BIP, F3, B3 and the loops (LF/LB); dNTPs (1.4 mM of each); Tris-HC1 buffer (20 mM); KC1 (50 mM); (NH4)SO4 (10 mM); MgSO4 (8 mM); Tween 20 (0.1%); DTT (1 mM); with viral RNA. The reactions are optimized with a maximum of 0.1 ng of viral RNA per reaction. The mixture is incubated at 65 °C/30-50 min.

It is important to emphasize that the LAMP amplification reaction takes place at the same time as reverse transcription (RT-LAMP one step) using Bst 3.0 DNA polymerase which also has the RNA as a template or in the presence of RTx and Bst 2.0 WarmSmart (New England Biolabs).

As indicated earlier in this report, a responsive WEB application with access on any user device with any available screen size, such as computer, tablet and smartphone, was developed for remote monitoring of the LAMP device of the invention. In this specific test, the application uses the JavaScript programming language, with ReactJS for the frontend and NodeJS for the backend. It has a Postgresql relational database to hold all the information, as well as a non-relational database such as MongoDB or Redis for quick consultations and management report processing.

The informations are transmitted encrypted to the server in AWS with a docker container system on Linux, allowing you to distribute the load of multiple accesses. In addition, all services provided through Telemedicine have the appropriate technological infrastructure, pertinent and obeying the security rules concerning storage, handling, data transmission, confidentiality, privacy, and guarantee of professional secrecy, according to the Brazilian legislation, CFM Resolution No. 1.643/2002.

The LAMP device of the present invention is a dedicated microprocessor-based computing device. The system is compatible with 10 microtubes 81 of 0,2mL. The heating required for isothermal amplification (about 65 °C) is achieved and stabilized by activating ceramic resistor wires. The lid will be independently heated up to about 75 °C, controlled by a PID (Proportional-Integrative-Derivative) algorithm. The working temperature range of the block will be 25°C to 70°C. The temperature control will be ensured by the use of thermistors and PID algorithm. The power supply is bivolt 100/240 V; frequency: 50-60 Hz; consumption: 70 W. The system is operated via microcontroller by Arduino UNO R3 (ATmega328) that communicates with the smartphone by a low-power HM11 Bluetooth module. One of the Arduino's digital outputs supplies 5 V input using MOSFETs. A temperature sensor monitors the heating and sends a signal to the control board with central processing. The analog reading is converted into a temperature that will be continuously monitored to operate the heating in an isothermal manner using pulse width modulation.

Samples collected by nasal and throat swabs from patients with suspected COVID-19 are collected and diluted in 2 mL of DMEM culture means and 3 IttL used to perform the isothermal amplification reaction on the device.

For the RT-LAMP reaction in 96-well plates, 12.5 zuL of the "WarmSmart Colorimetric Lamp 2X Master mix" reagent (New England Biolabs, M1800) are used in the presence of DEPC-treated ultrapure water and the SARS-CoV-2-specific primers. 3 zuL of sample containing RNA are used. Controls are used in the absence of RNA (negative) and internal reaction controls. The plate is incubated at 65°C for 30 min. Alternatively, the same process is repeated in the presence of a DNA intercalator, SYTO-9, to evaluate the quantitative profile using real-time PCR thermal cycler. The sensitivity and specificity of LAMP reactions are compared with RT-PCR.

The World Health Organization - WHO has recommended a large-scale testing for every suspected case of COVID-19 and immediate isolation for cases where testing is positive. Such actions are the main weapons in the fight against spreading the virus, since it makes possible to break the chain of transmission. Therefore, the improvement, expansion, and diagnostic speed in COVID are of strategic importance.

Regarding hospital care, the more tests are performed in hospitalized suspect patients, the more agile will become the provision of services, with reduction in the occupancy rate and greater availability of beds, medications, and respirators for the population, with direct impact on cost reduction and an economic and social efficiency. Patients with negative tests will not need to stay in isolation and will have another conduction of therapy, with no risk of exposure to the truly infected.

The diagnostic clarification also allows a better dimensioning of the pandemic, since currently the tests are being made available only for severe cases, generating an under dimensioning of the data of total cases of infection, and on the other hand an over dimensioning of the lethality rate, with a consequent undesirable bias in the epidemiological profile. The calculation of epidemiological data allows a better knowledge of the behavior of the virus in the local reality, subsidizing policies in Public Health.

In addition, the expansion in diagnostic capacity enables the optimization of health-related work processes for professionals all over the country. Doctors and nurses, directly involved in the care of suspected patients, can have more confidence in the management of cases through diagnostic clarification. The prospect of testing the health professionals themselves points to a significant reduction in absenteeism, and can minimize staff shortages in hospitals, with a consequent increase in the workload of those who work on the front line in cases that are negative for the virus.

The present invention presents great positive impact in fighting/controlling the global coronavirus pandemic faced by mankind nowadays.

It should be noted that numerous variations affecting the scope of protection of the present application are permissible. Thus, it is reinforced that the present invention is not limited to the particular configurations/embodiments described above.

## Claims

1. LAMP assay device that promotes isothermal RNA/DNA amplification applied to pathogen identification, **characterized by** comprising:
a LAMP assay chamber (1); an electronics cabinet (4), a power supply means (7); and a top lid (2) for closing the LAMP assay chamber (1),
wherein internally the device comprises: a metal cylindrical thermoblock (8) comprising openings (80) for positioning microtubes (81); a control board with central processing (14); a power electronic board (13); at least one heating element (10) in contact with the thermoblock (8) and adapted to heat the thermoblock (8) by induction; a temperature sensor (9) adapted to measure the temperature of the thermoblock (8); a plurality of RGB LEDs (12) positioned below the thermoblock (8) and adapted to excite each microtube positioned in the thermoblock (8); and a camera (11) positioned below the thermoblock (8) and adapted to capture images of each of the microtubes (81) positioned in the thermoblock (8).

2. LAMP assay device according to claim 1, **characterized by** the control board with central processing (14) comprising means for communicating with a mobile user device and adapted to control the temperature of the thermoblock (8) according to a schedule established by controlling the heating element (10) and in response to a signal from the temperature sensor (9).

3. LAMP assay device according to claim 1 or 2, **characterized by** comprising a visual operating warning element adapted to inform a user of the status of the device, wherein the visual element is an LED positioned externally to the device adapted to indicate via specific colors, the condition of the device.

4. LAMP assay device according to any of the claims 1 to 3, **characterized by** comprising a lid closing sensor (3) in communication with the central processing control board (14), wherein the central processing control board (14) is adapted to allow operation of the device only when the top lid (2) is closed.

5. LAMP assay device according to any of the claims 1 to 4, **characterized by** externally comprising a power source connected to a power entry point; a bottom cover (6); an electronics cabinet (4) in which the electronic control devices are positioned; and an on/off button (5).

6. LAMP assay device according to any of the claims 1 to 5, **characterized by** the thermoblock (8) comprising a cylindrical anodized aluminum block in which each opening is adapted to fairly accommodate a 0.2 mL microtube.

7. LAMP assay device according to any one of the claims 1 to 6, **characterized by** the heating element (10) in contact with the thermoblock (8) and heating the support by induction.

8. LAMP assay device according to any of the claims 1 to 7, **characterized by** the openings (80) for positioning microtubes (81) angled at an angle ranging from 38° to 52°.

9. LAMP assay device according to any of the claims 1 to 8, **characterized by** the thermoblock (8) comprising a hole (82) in the lower inner portion of each opening (80) for positioning microtubes (81), wherein the hole (82) is adapted to allow excitation of light with four bands of fluorescence signal, in addition to making it possible to image each microtube uniquely by the camera (11).

10. LAMP assay device according to any of the claims 1 to 9, **characterized by** the communication between the control board with central processing (14) and the user device is performed by wireless technology.

11. Method for identifying pathogens from a LAMP assay device as defined in any of the claims 1 to 10, **characterized by** comprising the steps of:
activate at least one heating element (10) to raise the temperature of the thermoblock (8) to a preset temperature for the defined diagnostic reaction;
maintain the temperature of the thermoblock (8) at the preset temperature for a preset period of time for the defined diagnostic reaction, and turn off the heating element (10);
turn on the plurality of RGB LEDs (12) for excitation of each microtube positioned in the thermoblock (8);
capture the values with camera image (11) for each position of each microtube and for each of the excitation colors, and send this information to the user device;
processing the image information captured by camera 11, which involves converting the image information from RGB to chrominance and checking in which region of the 2D plane each value is located;
define a positive or negative diagnosis for each microtube from the information processing; and
display the diagnostic result of each microtube through the user device.

12. Method, according to claim 11, **characterized by** comprising the step of performing a real-time reading using displaceable probes in constructing a fluorescence vs time curve.

13. Method, according to claim 11 or 12, **characterized by** comprising the steps of: storing the diagnostic result of each microtube in a local database; and sending the diagnostic result of each microtube to a cloud database.

14. Method according to any of the claims 11 to 13, **characterized by** the step of capturing values with camera imaging (11) for each position of each microtube, and for each of the excitation colors, further comprising using the green, red and orange fluorophores

15. Method, according to any of the claims 11 to 14, **characterized by** compriing a step of activating a cooling device to cool the assay chamber (1) of the LAMP device.

16. A method of diagnosis on a biological sample, **characterized by** the fact that it comprises:
(i) subject the sample to a loop-mediated isothermal amplification (LAMP) by using a primer set specific for the target nucleic acid, where the primer set comprises:
(a) six primers, each, respectively, having a sequence at least 90% identical to SEQ ID NO: 61, SEQ ID NO: 62, SEQ ID NO: 63, SEQ ID NO: 64, SEQ ID NO: 65, and SEQ ID NO: 66; or
(b) six primers, each, respectively, having a sequence at least 90% identical to SEQ ID NO: 67, SEQ ID NO: 68, SEQ ID NO: 69, SEQ ID NO: 70, SEQ ID NO: 71 and SEQ ID NO: 72, or
(c) six primers, each, respectively, having a sequence at least 90% identical to SEQ ID NO: 1, SEQ ID NO: 2, SEQ ID NO: 3, SEQ ID NO: 4, SEQ ID NO: 5, and SEQ ID NO: 6; or
(d) six primers each, respectively, having a sequence at least 90% identical to SEQ ID NO: 7, SEQ ID NO: 8, SEQ ID NO: 9, SEQ ID NO: 10, SEQ ID NO: 11, and SEQ ID NO: 12; or
(e) six primers, each, respectively, having a sequence at least 90% identical to SEQ ID NO: 13, SEQ ID NO: 14, SEQ ID NO: 15, SEQ ID NO: 16, SEQ ID NO: 17, and SEQ ID NO: 18; or
(f) six primers, each, respectively, having a sequence at least 90% identical to SEQ ID NO: 19, SEQ ID NO: 20, SEQ ID NO: 21, SEQ ID NO: 22, SEQ ID NO: 23, and SEQ ID NO: 24; or
(g) six primers, each, respectively, having a sequence at least 90% identical to SEQ ID NO: 25, SEQ ID NO: 26, SEQ ID NO: 27, SEQ ID NO: 28, SEQ ID NO: 29, and SEQ ID NO: 30; or
(h) six primers, each, respectively, having a sequence at least 90% identical to SEQ ID NO: 31, SEQ ID NO: 32, SEQ ID NO: 33, SEQ ID NO: 34, SEQ ID NO: 35, and SEQ ID NO: 36; or
(i) six primers, each, respectively, having a sequence at least 90% identical to SEQ ID NO: 37, SEQ ID NO: 38, SEQ ID NO: 39, SEQ ID NO: 40, SEQ ID NO: 41, and SEQ ID NO: 42; or
(j) six primers, each, respectively, having a sequence at least 90% identical to SEQ ID NO: 43, SEQ ID NO: 44, SEQ ID NO: 45, SEQ ID NO: 46, SEQ ID NO: 47, and SEQ ID NO: 48; or
(k) six primers, each, respectively, having a sequence at least 90% identical to SEQ ID NO: 49, SEQ ID NO: 50, SEQ ID NO: 51, SEQ ID NO: 52, SEQ ID NO: 53, and SEQ ID NO: 54; or
(l) six primers, each, respectively, having a sequence at least 90% identical to SEQ ID NO: 55, SEQ ID NO: 56, SEQ ID NO: 57, SEQ ID NO: 58, SEQ ID NO: 59 and SEQ ID NO: 60; or
(m) a combination of (a) and (b); or
(n) the combination of (c) and (d); or
(o) the combination of (f) and (g); or
(p) the combination of (h) and (i); or
(q) the combination of (j) and (k); and
(ii) detect the target nucleic acid amplification product in the biological sample.

17. Method according to claim 16, **characterized by** step (i) the method is an RT-LAMP.

18. Method according to claim 16 or 17, **characterized by** step (i) the target nucleic acid is a nucleic acid of SARS-CoV2.

19. Method according to claim 18, **characterized by** the fact that, in step (i), the primer sets comprise:
(a) six primers, each, respectively, having a sequence at least 90% identical to SEQ ID NO: 25, SEQ ID NO: 26, SEQ ID NO: 27, SEQ ID NO: 28, SEQ ID NO: 29, and SEQ ID NO: 30; or
(b) six primers, each, respectively, having a sequence at least 90% identical to SEQ ID NO: 31, SEQ ID NO: 32, SEQ ID NO: 33, SEQ ID NO: 34, SEQ ID NO: 35, and SEQ ID NO: 36; or
(c) six primers, each, respectively, having a sequence at least 90% identical to SEQ ID NO: 37, SEQ ID NO: 38, SEQ ID NO: 39, SEQ ID NO: 40, SEQ ID NO: 41, SEQ ID NO: 42; or
(d) six primers, each, respectively, having a sequence at least 90% identical to SEQ ID NO: 43, SEQ ID NO: 44, SEQ ID NO: 45, SEQ ID NO: 46, SEQ ID NO: 47, and SEQ ID NO: 48; or
(e) six primers, each, respectively, having a sequence at least 90% identical to SEQ ID NO: 49, SEQ ID NO: 50, SEQ ID NO: 51, SEQ ID NO: 52, SEQ ID NO: 53, and SEQ ID NO: 54; or
(f) six primers, each, respectively, having a sequence at least 90% identical to SEQ ID NO: 55, SEQ ID NO: 56, SEQ ID NO: 57, SEQ ID NO: 58, SEQ ID NO: 59 and SEQ ID NO: 60; or
(g) six primers, each, respectively, having a sequence at least 90% identical to SEQ ID NO: 61, SEQ ID NO: 62, SEQ ID NO: 63, SEQ ID NO: 64, SEQ ID NO: 65, and SEQ ID NO: 66; or
(h) six primers, each, respectively, having a sequence at least 90% identical to SEQ ID NO: 67, SEQ ID NO: 68, SEQ ID NO: 69, SEQ ID NO: 70, SEQ ID NO: 71 and SEQ ID NO: 72; or
(i) a combination of (a) and (b); or
(j) the combination of (c) and (d).

20. Method according to any of the claims 16 to 19, **characterized by** the LAMP reaction performed at a temperature in the range of about 60°C to 70°C.

21. Method according to any of the claims 16 to 20, **characterized by** the fact that the RT-LAMP reaction includes a pH-sensitive indicator dye.

22. Method according to any of the claims 16 to 21, characterizedby the fact that the pH sensitive indicator dye is a colored dye detectable in visible light.

23. Method according to any of the claims 16 to 20, **characterized by** the detection performed using fluorescently displaced probes.

24. A diagnostic kit on a biological sample, **characterized by** the fact that it comprises the primer sets, comprising:
(a) six primers, each, respectively, having a sequence at least 90% identical to SEQ ID NO: 61, SEQ ID NO: 62, SEQ ID NO: 63, SEQ ID NO: 64, SEQ ID NO: 65, and SEQ ID NO: 66; or
(b) six primers, each, respectively, having a sequence at least 90% identical to SEQ ID NO: 67, SEQ ID NO: 68, SEQ ID NO: 69, SEQ ID NO: 70, SEQ ID NO: 71, and SEQ ID NO: 72; or
(c) six primers, each, respectively, having a sequence at least 90% identical to SEQ ID NO: 1, SEQ ID NO: 2, SEQ ID NO: 3, SEQ ID NO: 4, SEQ ID NO: 5, and SEQ ID NO: 6; or
(d) six primers each, respectively, having a sequence at least 90% identical to SEQ ID NO: 7, SEQ ID NO: 8, SEQ ID NO: 9, SEQ ID NO: 10, SEQ ID NO: 11, and SEQ ID NO: 12; or
(e) six primers, each, respectively, having a sequence at least 90% identical to SEQ ID NO: 13, SEQ ID NO: 14, SEQ ID NO: 15, SEQ ID NO: 16, SEQ ID NO: 17, and SEQ ID NO: 18; or
(f) six primers, each, respectively, having a sequence at least 90% identical to SEQ ID NO: 19, SEQ ID NO: 20, SEQ ID NO: 21, SEQ ID NO: 22, SEQ ID NO: 23, and SEQ ID NO: 24; or
(g) six primers, each, respectively, having a sequence at least 90% identical to SEQ ID NO: 25, SEQ ID NO: 26, SEQ ID NO: 27, SEQ ID NO: 28, SEQ ID NO: 29, and SEQ ID NO: 30; or
(h) six primers, each, respectively, having a sequence at least 90% identical to SEQ ID NO: 31, SEQ ID NO: 32, SEQ ID NO: 33, SEQ ID NO: 34, SEQ ID NO: 35, and SEQ ID NO: 36; or
(i) six primers, each, respectively, having a sequence at least 90% identical to SEQ ID NO: 37, SEQ ID NO: 38, SEQ ID NO: 39, SEQ ID NO: 40, SEQ ID NO: 41, and SEQ ID NO: 42; or
(j) six primers, each, respectively, having a sequence at least 90% identical to SEQ ID NO: 43, SEQ ID NO: 44, SEQ ID NO: 45, SEQ ID NO: 46, SEQ ID NO: 47, and SEQ ID NO: 48; or
(k) six primers, each, respectively, having a sequence at least 90% identical to SEQ ID NO: 49, SEQ ID NO: 50, SEQ ID NO: 51, SEQ ID NO: 52, SEQ ID NO: 53, and SEQ ID NO: 54; or
(l) six primers, each, respectively, having a sequence at least 90% identical to SEQ ID NO: 55, SEQ ID NO: 56, SEQ ID NO: 57, SEQ ID NO: 58, SEQ ID NO: 59 and SEQ ID NO: 60; or
(m) a combination of (a) and (b); or
(n) the combination of (c) and (d); or
(p) the combination of (f) and (g); or
(q) the combination of (h) and (i); or
(r) the combination of (j) and (k).

25. Oligonucleotide, **characterized by** having a sequence as defined in SEQ ID NO: 1 to SEQ ID NO: 72, or a sequence having 90% identity therewith.

## Amended claims

### Amended claims under Art. 19.1 PCT

1. LAMP assay device that promotes isothermal RNA/DNA amplification applied to pathogen identification, **characterized by** comprising:
a LAMP assay chamber (1); an electronics cabinet (4), a power supply means (7); and a top lid (2) for closing the LAMP assay chamber (1),
wherein internally the device comprises: a metal cylindrical thermoblock (8) comprising openings (80) for positioning microtubes (81); a control board with central processing (14); a power electronic board (13); at least one heating element (10) in contact with the thermoblock (8) and adapted to heat the thermoblock (8) by induction; a temperature sensor (9) adapted to measure the temperature of the thermoblock (8); a plurality of RGB LEDs (12) positioned below the thermoblock (8) and adapted to excite each microtube positioned in the thermoblock (8); and a camera (11) positioned below the thermoblock (8) and adapted to capture images of each of the microtubes (81) positioned in the thermoblock (8).

2. LAMP assay device according to claim 1, **characterized by** the control board with central processing (14) comprising means for communicating with a mobile user device and adapted to control the temperature of the thermoblock (8) according to a schedule established by controlling the heating element (10) and in response to a signal from the temperature sensor (9).

3. LAMP assay device according to claim 1 or 2, **characterized by** comprising a visual operating warning element adapted to inform a user of the status of the device, wherein the visual element is an LED positioned externally to the device adapted to indicate via specific colors, the condition of the device.

4. LAMP assay device according to any of the claims 1 to 3, **characterized by** comprising a lid closing sensor (3) in communication with the central processing control board (14), wherein the central processing control board (14) is adapted to allow operation of the device only when the top lid (2) is closed.

5. LAMP assay device according to any of the claims 1 to 4, **characterized by** externally comprising a power source connected to a power entry point; a bottom cover (6); an electronics cabinet (4) in which the electronic control devices are positioned; and an on/off button (5).

6. LAMP assay device according to any of the claims 1 to 5, **characterized by** the thermoblock (8) comprising a cylindrical anodized aluminum block in which each opening is adapted to fairly accommodate a 0.2 mL microtube.

7. LAMP assay device according to any one of the claims 1 to 6, **characterized by** the heating element (10) in contact with the thermoblock (8) and heating the support by induction.

8. LAMP assay device according to any of the claims 1 to 7, **characterized by** the openings (80) for positioning microtubes (81) angled at an angle ranging from 38° to 52°.

9. LAMP assay device according to any of the claims 1 to 8, **characterized by** the thermoblock (8) comprising a hole (82) in the lower inner portion of each opening (80) for positioning microtubes (81), wherein the hole (82) is adapted to allow excitation of light with four bands of fluorescence signal, in addition to making it possible to image each microtube uniquely by the camera (11).

10. LAMP assay device according to any of the claims 1 to 9, **characterized by** the communication between the control board with central processing (14) and the user device is performed by wireless technology.

11. Method for identifying pathogens from a LAMP assay device as defined in any of the claims 1 to 10, **characterized by** comprising the steps of:
activate at least one heating element (10) to raise the temperature of the thermoblock (8) to a preset temperature for the defined diagnostic reaction;
maintain the temperature of the thermoblock (8) at the preset temperature for a preset period of time for the defined diagnostic reaction, and turn off the heating element (10);
turn on the plurality of RGB LEDs (12) for excitation of each microtube positioned in the thermoblock (8);
capture the values with camera image (11) for each position of each microtube and for each of the excitation colors, and send this information to the user device;
processing the image information captured by camera 11, which involves converting the image information from RGB to chrominance and checking in which region of the 2D plane each value is located;
define a positive or negative diagnosis for each microtube from the information processing; and
display the diagnostic result of each microtube through the user device.

12. Method, according to claim 11, **characterized by** comprising the step of performing a real-time reading using displaceable probes in constructing a fluorescence vs time curve.

13. Method, according to claim 11 or 12, **characterized by** comprising the steps of: storing the diagnostic result of each microtube in a local database; and sending the diagnostic result of each microtube to a cloud database.

14. Method according to any of the claims 11 to 13, **characterized by** the step of capturing values with camera imaging (11) for each position of each microtube, and for each of the excitation colors, further comprising using the green, red and orange fluorophores

15. Method, according to any of the claims 11 to 14, **characterized by** comprising a step of activating a cooling device to cool the assay chamber (1) of the LAMP device.

16. A method of diagnosis on a biological sample, **characterized by** the fact that it comprises:
(i) subject the sample to a loop-mediated isothermal amplification (LAMP) by using a primer set specific for the target nucleic acid, where the primer set comprises:
(a) six primers, each, respectively, having a sequence at least 90% identical to SEQ ID NO: 61, SEQ ID NO: 62, SEQ ID NO: 63, SEQ ID NO: 64, SEQ ID NO: 65, and SEQ ID NO: 66; or
(b) six primers, each, respectively, having a sequence at least 90% identical to SEQ ID NO: 67, SEQ ID NO: 68, SEQ ID NO: 69, SEQ ID NO: 70, SEQ ID NO: 71 and SEQ ID NO: 72, or
(c) six primers, each, respectively, having a sequence at least 90% identical to SEQ ID NO: 1, SEQ ID NO: 2, SEQ ID NO: 3, SEQ ID NO: 4, SEQ ID NO: 5, and SEQ ID NO: 6; or
(d) six primers each, respectively, having a sequence at least 90% identical to SEQ ID NO: 7, SEQ ID NO: 8, SEQ ID NO: 9, SEQ ID NO: 10, SEQ ID NO: 11, and SEQ ID NO: 12; or
(e) six primers, each, respectively, having a sequence at least 90% identical to SEQ ID NO: 13, SEQ ID NO: 14, SEQ ID NO: 15, SEQ ID NO: 16, SEQ ID NO: 17, and SEQ ID NO: 18; or
(f) six primers, each, respectively, having a sequence at least 90% identical to SEQ ID NO: 19, SEQ ID NO: 20, SEQ ID NO: 21, SEQ ID NO: 22, SEQ ID NO: 23, and SEQ ID NO: 24; or
(g) six primers, each, respectively, having a sequence at least 90% identical to SEQ ID NO: 25, SEQ ID NO: 26, SEQ ID NO: 27, SEQ ID NO: 28, SEQ ID NO: 29, and SEQ ID NO: 30; or
(h) six primers, each, respectively, having a sequence at least 90% identical to SEQ ID NO: 31, SEQ ID NO: 32, SEQ ID NO: 33, SEQ ID NO: 34, SEQ ID NO: 35, and SEQ ID NO: 36; or
(i) six primers, each, respectively, having a sequence at least 90% identical to SEQ ID NO: 37, SEQ ID NO: 38, SEQ ID NO: 39, SEQ ID NO: 40, SEQ ID NO: 41, and SEQ ID NO: 42; or
(j) six primers, each, respectively, having a sequence at least 90% identical to SEQ ID NO: 43, SEQ ID NO: 44, SEQ ID NO: 45, SEQ ID NO: 46, SEQ ID NO: 47, and SEQ ID NO: 48; or
(k) six primers, each, respectively, having a sequence at least 90% identical to SEQ ID NO: 49, SEQ ID NO: 50, SEQ ID NO: 51, SEQ ID NO: 52, SEQ ID NO: 53, and SEQ ID NO: 54; or
(l) six primers, each, respectively, having a sequence at least 90% identical to SEQ ID NO: 55, SEQ ID NO: 56, SEQ ID NO: 57, SEQ ID NO: 58, SEQ ID NO: 59 and SEQ ID NO: 60; or
(m) a combination of (a) and (b); or
(n) the combination of (c) and (d); or
(o) the combination of (f) and (g); or
(p) the combination of (h) and (i); or
(q) the combination of (j) and (k); and
(ii) detect the target nucleic acid amplification product in the biological sample.

17. Method according to claim 16, **characterized by** step (i) the method is an RT-LAMP.

18. Method according to claim 16 or 17, **characterized by** step (i) the target nucleic acid is a nucleic acid of SARS-CoV2.

19. Method according to claim 18, **characterized by** the fact that, in step (i), the primer sets comprise:
(a) six primers, each, respectively, having a sequence at least 90% identical to SEQ ID NO: 25, SEQ ID NO: 26, SEQ ID NO: 27, SEQ ID NO: 28, SEQ ID NO: 29, and SEQ ID NO: 30; or
(b) six primers, each, respectively, having a sequence at least 90% identical to SEQ ID NO: 31, SEQ ID NO: 32, SEQ ID NO: 33, SEQ ID NO: 34, SEQ ID NO: 35, and SEQ ID NO: 36; or
(c) six primers, each, respectively, having a sequence at least 90% identical to SEQ ID NO: 37, SEQ ID NO: 38, SEQ ID NO: 39, SEQ ID NO: 40, SEQ ID NO: 41, SEQ ID NO: 42; or
(d) six primers, each, respectively, having a sequence at least 90% identical to SEQ ID NO: 43, SEQ ID NO: 44, SEQ ID NO: 45, SEQ ID NO: 46, SEQ ID NO: 47, and SEQ ID NO: 48; or
(e) six primers, each, respectively, having a sequence at least 90% identical to SEQ ID NO: 49, SEQ ID NO: 50, SEQ ID NO: 51, SEQ ID NO: 52, SEQ ID NO: 53, and SEQ ID NO: 54; or
(f) six primers, each, respectively, having a sequence at least 90% identical to SEQ ID NO: 55, SEQ ID NO: 56, SEQ ID NO: 57, SEQ ID NO: 58, SEQ ID NO: 59 and SEQ ID NO: 60; or
(g) six primers, each, respectively, having a sequence at least 90% identical to SEQ ID NO: 61, SEQ ID NO: 62, SEQ ID NO: 63, SEQ ID NO: 64, SEQ ID NO: 65, and SEQ ID NO: 66; or
(h) six primers, each, respectively, having a sequence at least 90% identical to SEQ ID NO: 67, SEQ ID NO: 68, SEQ ID NO: 69, SEQ ID NO: 70, SEQ ID NO: 71 and SEQ ID NO: 72; or
(i) a combination of (a) and (b); or
(j) the combination of (c) and (d).

20. Method according to any of the claims 16 to 19, **characterized by** the LAMP reaction performed at a temperature in the range of about 60°C to 70°C.

21. Method according to any of the claims 16 to 20, **characterized by** the fact that the RT-LAMP reaction includes a pH-sensitive indicator dye.

22. Method according to any of the claims 16 to 21, characterizedby the fact that the pH sensitive indicator dye is a colored dye detectable in visible light.

23. Method according to any of the claims 16 to 20, **characterized by** the detection performed using fluorescently displaced probes.

24. A diagnostic kit on a biological sample, **characterized by** the fact that it comprises the primer sets, comprising:
(a) six primers, each, respectively, having a sequence at least 90% identical to SEQ ID NO: 61, SEQ ID NO: 62, SEQ ID NO: 63, SEQ ID NO: 64, SEQ ID NO: 65, and SEQ ID NO: 66; or
(b) six primers, each, respectively, having a sequence at least 90% identical to SEQ ID NO: 67, SEQ ID NO: 68, SEQ ID NO: 69, SEQ ID NO: 70, SEQ ID NO: 71, and SEQ ID NO: 72; or
(c) six primers, each, respectively, having a sequence at least 90% identical to SEQ ID NO: 1, SEQ ID NO: 2, SEQ ID NO: 3, SEQ ID NO: 4, SEQ ID NO: 5, and SEQ ID NO: 6; or
(d) six primers each, respectively, having a sequence at least 90% identical to SEQ ID NO: 7, SEQ ID NO: 8, SEQ ID NO: 9, SEQ ID NO: 10, SEQ ID NO: 11, and SEQ ID NO: 12; or
(e) six primers, each, respectively, having a sequence at least 90% identical to SEQ ID NO: 13, SEQ ID NO: 14, SEQ ID NO: 15, SEQ ID NO: 16, SEQ ID NO: 17, and SEQ ID NO: 18; or
(f) six primers, each, respectively, having a sequence at least 90% identical to SEQ ID NO: 19, SEQ ID NO: 20, SEQ ID NO: 21, SEQ ID NO: 22, SEQ ID NO: 23, and SEQ ID NO: 24; or
(g) six primers, each, respectively, having a sequence at least 90% identical to SEQ ID NO: 25, SEQ ID NO: 26, SEQ ID NO: 27, SEQ ID NO: 28, SEQ ID NO: 29, and SEQ ID NO: 30; or
(h) six primers, each, respectively, having a sequence at least 90% identical to SEQ ID NO: 31, SEQ ID NO: 32, SEQ ID NO: 33, SEQ ID NO: 34, SEQ ID NO: 35, and SEQ ID NO: 36; or
(i) six primers, each, respectively, having a sequence at least 90% identical to SEQ ID NO: 37, SEQ ID NO: 38, SEQ ID NO: 39, SEQ ID NO: 40, SEQ ID NO: 41, and SEQ ID NO: 42; or
(j) six primers, each, respectively, having a sequence at least 90% identical to SEQ ID NO: 43, SEQ ID NO: 44, SEQ ID NO: 45, SEQ ID NO: 46, SEQ ID NO: 47, and SEQ ID NO: 48; or
(k) six primers, each, respectively, having a sequence at least 90% identical to SEQ ID NO: 49, SEQ ID NO: 50, SEQ ID NO: 51, SEQ ID NO: 52, SEQ ID NO: 53, and SEQ ID NO: 54; or
(l) six primers, each, respectively, having a sequence at least 90% identical to SEQ ID NO: 55, SEQ ID NO: 56, SEQ ID NO: 57, SEQ ID NO: 58, SEQ ID NO: 59 and SEQ ID NO: 60; or
(m) a combination of (a) and (b); or
(n) the combination of (c) and (d); or
(p) the combination of (f) and (g); or
(q) the combination of (h) and (i); or
(r) the combination of (j) and (k).

25. Oligonucleotide, **characterized by** having a sequence as defined in SEQ ID NO: 1 to SEQ ID NO: 72, or a sequence having 90% identity therewith.
